Europäisches Patentamt

**European Patent Office**

**Office européen des brevets**

(19)

(11) Publication number: **0 111 293**
A2

(12)

# EUROPEAN PATENT APPLICATION

(21) Application number: 83112260.1

(22) Date of filing: 06.12.83

(51) Int. Cl.³: **C 12 P 13/20**
C 12 N 1/20, C 12 N 15/00
//C12R1/43

(30) Priority: 08.12.82 JP 215889/82

(43) Date of publication of application:
20.06.84 Bulletin 84/25

(84) Designated Contracting States:
DE FR GB

(71) Applicant: Tanabe Seiyaku Co., Ltd.
No. 21 Dosho-machi 3-chome Higashi-ku
Osaka-shi Osaka-fu(JP)

(72) Inventor: Chibata, Ichiro
No. 13-2, Fujishirodai 4-chome
Suita-shi Osaka-fu(JP)

(72) Inventor: Kisumi, Masahiko
No. 12-20, Morikitacho 4-chome Higashinada-ku
Kobe-shi Hyogo-ken(JP)

(72) Inventor: Nishimura, Noriyuki
No. 5-21, Uozaki-Nakamachi 3-chome Higashinada-ku
Kobe-shi Hyogo-ken(JP)

(74) Representative: Hansen, Bernd, Dr.rer.nat. et al,
Hoffmann, Eitle & Partner Patentanwälte Arabellastrasse
4
D-8000 München 81(DE)

(54) Microorganism of the genus Serratia, method of producing the same and method for producing L-aspartic acid.

(57) A microorganism of the genus *Serratia* having high aspartase activity and being released from catabolite repression and a method for producing such a microorganism comprising mutagenizing a parent strain of the genus *Serratia* having aspartase activity and being subjected to catabolite repression, cultivating the treated microorganism in a medium containing L-aspartic acid either as a sole nitrogen source or as sole carbon and nitrogen sources and isolating a microorganism being able to grow in the medium. A method for producing L-aspartic acid comprising contacting a culture of the microorganism, microbial cells collected from the culture or a processed material of the microbial cells with fumaric acid and ammonia to produce L-aspartic acid and then collecting L-aspartic acid thus produced is also provided.

EP 0 111 293 A2

Croydon Printing Company Ltd.

Microorganism of the genus Serratia, method of producing
the same and method for producing L-aspartic acid

The present invention relates to a novel microorganism useful for production of L-aspartic acid, a method
for producing such a novel microorganism and a method for
producing L-aspartic acid using the microorganism.

It has hitherto been known that Serratia
marcescens has aspartase activity, and a method of enzymatic
production of L-aspartic acid from ammonium fumarate (or
fumaric acid and an ammonium salt) by using such a
microorganism has been also known in the prior art (Japanese
Patent Publication No. 12553/1979). However, aspartase
activity of Serratia marcescens used in such a conventional
method is not so high as it is sufficient for industrial
production of L-aspartic acid. In addition, it has been
known that, in the presence of a readily assimilable carbon
source such as glucose, formation of an enzyme responsible
for utilization of another carbon source in Serratia
marcescens having aspartase activity is repressed, i.e. the
microorganism is subjected to so-called catabolite
repression [Biochem. J., 36, 619 (1942)]. Therefore, when
cultivation of Serratia marcescens is carried out by
addition of a readily assimilable carbon source such as
glucose to a medium, aspartase activity of the microorganism
is insufficiently high and, hence, the yield of L-aspartic
acid is decreased.

Under these circumstances, in order to establish
an industrially advantageous process for producing

L-aspartic acid, the present inventors have intensively studied. As the result, the present inventors have found that mutants having very high aspartase activity in comparison with that of their parent strain and being released from catabolite repression can be obtained by treating a microorganism of the genus Serratia to induce mutation and cultivating the treated microorganism in a certain medium and can be advantageously utilized in the production of L-aspartic acid in an industrial scale.

One object of the present invention is to provide a novel microorganism useful for industrial production of L-aspartic acid.

Another object of the present invention is to provide a method for production such a novel microorganism.

Still, another object of the present invention is to provide an industrially advantageous method for producing L-aspartic acid. These objects as well as other objects and advantages of the present invention will become apparent to those skilled in the art from the following description.

According to the present invention, there is provided a novel microorganism of the genus Serratia having high aspartase activity and being released from catabolite repression and a method for producing such a novel microorganism comprising mutagenizing a microorganism of the genus Serratia having aspartase activity and being subjected to catabolite repression, cultivating the treated microorganism in a medium containing L-aspartic acid either as a sole nitrogen source or as sole carbon and nitrogen sources and collecting a microorganism being able to grow in the medium.   A method for producing L-aspartic acid comprising contacting a culture of the novel microorganism, microbial cells collected from the culture or a processed material of the microbial cells with fumaric acid and ammonia to produce L-aspartic acid and then collecting L-aspartic acid thus produced is also provided.

The novel microorganism of the genus _Serratia_ having high aspartase activity and being released from catabolite repression of the present invention can be obtained by mutagenizing a parent strain, i.e., a microorganism of the genus _Serratia_ having aspartase activity and being subjected to catabolite repression, cultivating the treated microorganism in a medium containing L-aspartic acid either as a sole nitrogen source or as sole carbon and nitrogen sources to isolate a strain which can grow faster than the parent strain in the medium.

The microorganism to be used as the parent strain is not limited to a specific one so far as it is a microorganism of the genus _Serratia_ having aspartase activity.  A suitable examples of the parent strain are _Serratia marcescens_ SR41 (FERM P-7014, Fermentation Research Institute (FRI), Agency of Industrial Science and Technology, Ibaragi-ken, Japan) and _Serratia marcescens_, deposition number OUT 8259 (Osaka University in Osaka/Japan).

The mutagenesis of the parent strain can be carried out according to a conventional method. For example, the treatment with N-methyl-N'-nitro-N-nitrosoguanidine or ultraviolet radiation can be employed.

The concentration of L-aspartic acid in a medium used for cultivation of the microorganism treated by the mutagenesis is preferably about 0.005 to 5 % regardless of using L-aspartic acid either as a sole nitrogen source or as sole carbon and nitrogen sources. When L-aspartic acid is used as a sole nitrogen source, glucose, sucrose or the like can be used as a carbon source. For example, when glucose is used, its concentration in the medium is preferably about 0.1 to 10 %. Further, in both cases of using L-aspartic acid as a sole nitrogen source and using it as sole carbon and nitrogen sources, various salts, trace elements and the like used for a conventional nutrient medium can be added to the medium.

The method for cultivating the microorganism treated by the mutagenesis is not limited to a specific one and the cultivation can be carried out according to a conventional method. For example, a so-called continuous cultivation method ["Saikin, Phage Jikken-ho" (the separate volume of Tampakushitsu, Kakusan, Koso), p 35 (1972)] can be suitably employed. In such a method, the microorganism treated by the mutagenesis is inoculated in a flask containing a medium and the flask is incubated at 10 to 45°C, preferably at 28 to 37°C with continuously pouring the additional medium into the flask at a constant rate of addition (e.g., degree of dilution: 0.05 to 0.5/hr), while the equal volume of the resulting culture is removed from the flask to concentrate and separate microbial cells which can quickly grow under such conditions. Then, the culture thus obtained is spread on plates containing a medium having the same composition as that of the medium used in the

0111293

continuous cultivation but containing about 0.1 to 5 % of L-aspartic acid and cultivated at 10 to 45°C, preferably at 28 to 37°C for 1 to 3 days. A large colony grown on the plates is isolated to give the desired microorganism having high aspartase activity such as 9 to 13 $\mu$mole/min/mg protein as measured by the method disclosed hereinafter and being released from catabolite repression.

The representatives of the microorganism having high aspartase activity and being released from catabolite repression thus obtained are deposited with Fermentation Research Institute (FRI), Agency of Industrial Science and Technology, Ibaragi-ken, Japan under Budapest Treaty on November 13, 1982. They are Serratia marcescens APc-109 (FERM BP-391) and Serratia marcescens APc-494 (FERM BP-392). Aspartase activities of these microorganisms are about 4 times as high as that of the parent strain or more. In addition, the microorganism of the present invention has such a characteristic that it has very high enzymatic activities responsible for tricarboxylic acid cycle (TCA cycle) such as citric acid synthase, $\alpha$-ketoglutarate dehydrogenase complex and fumarase activities as well as very high glutamate dehydrogenase and D-serine deaminase activities in comparison with those of the parent strain.

Since, as described in the above, aspartase activity of the microorganism of the present invention is extremely superior to that of the parent strain, L-aspartic acid can be advantageously from fumaric acid and ammonia by using the microorganism.

That is, L-aspartic acid can be produced by contacting a culture of the microorganism, microbial cells collected from the culture or a processed material of the microbial cells with the substrates, i.e., fumaric acid and ammonia to effect L-aspartic acid-producing enzymatic reaction.

The microorganism of the present invention can be cultivated by using a conventional nutrient medium containing carbon sources (e.g., glucose, sucrose, glycerol, fumaric acid), nitrogen sources (e.g., ammonium fumarate, ammonium sulfate), organic nutrients (e.g., yeast extract, peptone, corn steep liquor, meat extract), inorganic salts (e.g., potassium dihydrogen phosphate, magnesium sulfate), and the like. The cultivation can be carried out according to a conventional method. For example, after adjusting pH of a medium to 5.0 to 9.0, the microorganism is inoculated into the medium and cultivated at 10 to 45 °C, preferably, at 28 to 37°C for 12 to 92 hours under aerobic conditions. In the medium, L-aspartic acid can be also used as a carbon source as well as a nitrogen source and the preferred amount thereof is about 0.1 to 5 %.

As described in the above, when L-aspartic acid-producing reaction is carried out, not only the culture of the microorganism but also microbial cells collected from the culture and a processed material of the microbial cells

- 7 -

can be used.

The collection of the microbial cells from the culture can be carried out according to a conventional method such as filtration, centrifugation, and the like.

Examples of the processed material of the microbial cells are washed microbial cells prepared by washing the microbial cells with physiological saline solution, phosphate buffer solution, carbonate buffer solution and the like; dried microbial cells prepared by drying the microbial cells according to a conventional method such as lyophilization, acetone treatment and the like; ground microbial cells prepared by grinding the microbial cells with glass beads, alumina and the like; autolysates of the microbial cells prepared by toluene, chloroform and the like; sonicates of the microbial cells prepared by sonic oscillator microbial cell extracts prepared by french press; and immobilized preparations obtained by immobilizing the microbial cells or the above other processed materials thereof according to a known method such as gel conjugation method, adsorption method or the like. Examples of the immobilized preparations are those obtained by immobilizing the microbial cells or the above other processed materials thereof on supports, carriers or bases such as polyacrylamide gel, sulfur-containing polysaccharide (e.g.,

carrageenan, furcellaran, etc.) gel, collagen gel, alginic acid gel, polyvinyl alcohol gel, agar gel and the like. When polyacrylamide gel is used, the immobilization can be carried out according to the method disclosed in Japanese Patent Publication No. 1831/1978. When sulfur-containing polysaccharide gel is used, the method disclosed in Japanese Patent Laid Open Publication No. 6483/1978 can be employed. Further, when collagen gel, alginic acid gel, polyvinyl alcohol gel, agar gel or the like is used, the immobilization can be carried out according to the methods disclosed in Japanese Patent Laid Open Publication Nos. 144780/1976, 30582/1974, 80285/1974 and 133484/1976.

Fumaric acid and ammonia to be used as the substrates can be introduced into a L-aspartic acid-producing enzymatic reaction system in various forms. For example, they can be introduced into the reaction system in the form of ammonium fumarate or as fumaric acid or its salt and an inorganic ammonium salt.

As the salt of fumaric acid, for example, sodium fumarate or potassium fumarate can be suitably used. As the inorganic ammonium salt, for example, ammonium chloride, ammonium sulfate, ammonium phosphate or a mixture thereof can be suitably used.

In case of using fumaric acid or its salt and the inorganic ammonium salt, it is preferable that the molar ratio of these two components are within the range between 1 : 1.5 to 1 : 2.

The enzymatic reaction can be carried out at such a wide temperature range as about 5 to 50°C but, in view of stability of the enzyme of the microorganism, it is preferable that the enzymatic reaction is carried out at 20 to 45°C. Further, it is preferable to carry out the enzymatic reaction at a pH range of 6 to 10. Besides, when the enzymatic reaction is carried out, it is preferable to add a divalent metal ion such as calcium ion, magnesium ion, manganese ion, strontium ion or the like to the reaction system. The amount of the divalent metal ion can be about 0.1 to 10 mM and thereby stability of the enzyme can be improved.

When the microbial cells are used, preferably, the reaction is carried out in batchwise and L-aspartic acid can be produced by suspending the microbial cells collected from the culture thereof in a solution of the above substrates and stirring the suspension. When the immobilized preparation is used, the reaction can be carried out not only by a batch process but also by a continuous process using a column packed with the immobilized preparation since the immobilized preparation is insoluble in water. For example, the immobilized preparation is packed in a column and a substrate solution is passed down through the column at a suitable flow rate to obtain effluent containing only L-aspartic acid. When the reaction is carried out by a batch process, L-aspartic acid can be produced by suspending the immobilized preparation in a substrate solution and stirring the suspension. In the latter case, the immobilized preparation can be reused by separating it from the reaction mixture according to a standard method such as filtration or centrifugation. The progress of the above reaction is effected by the amount of microbial cells, temperature, reaction time, flow rate of the substrate (particularly, linear velocity) and the like. Optimal reaction conditions which can attain 100 % progress of the reaction can be readily found, for example, by suitably adjusting the flow rate of a substrate solution passing down through a column according to the amount of an immobilized preparation, in case of a continuous process using a column, or by suitably adjusting a reaction time, in case of a batch process.

L-Aspartic acid thus produced and accumulated in the reaction mixture can be readily separated and purified according to known methods, for example, by combining a conventional method using an ion exchange resin with other known methods.

As described hereinbefore, according to the present invention, the microorganism of the genus _Serratia_ having extremely high aspartase activity in comparison with that of a known microorganism of the genus _Serratia_ used in a conventional method for producing L-aspartic acid can be obtained. Further, according to the present invention, L-aspartic acid can be produced from fumaric acid and ammonia in a very high yield within a short period of time by using the microorganism of the genus _Serratia_ having such a high aspartase activity. With respect to the increase in aspartase activity of the microorganism of the present invention, although any specific mechanism has not yet been clarified, it is assumed that a gene thereof responsible for the production of aspartase is probably mutated. In addition, the microorganism of the present invention have such a characteristic that its aspartase activity is not decreased even in the presence of a readily assimilable carbon source such as glucose since the microorganism is released from catabolite repression. In other words, the aspartase activity of the microorganism is scarcely effected by the kinds of carbon sources to be used and the high aspartase activity is constantly revealed. Thereby, there is no need to pay attention to the kind of a carbon source used in the production of L-aspartic acid. This is one of the preferable characteristics of the present invention.

Furthermore, in addition to the high aspartase activity, the microorganism of the present invention has such a characteristic that all of the activities of the enzymes responsible for TCA cycle such as citric acid synthase, $\alpha$-ketoglutarate dehydrogenase complex and fumarase as well as glutamate dehydrogenase and D-serine deaminase in the presence of glucose are also extremely increased. Such a microorganism as activities of all enzymes thereof being increased has not hitherto been known in the prior art.

The following examples further illustrate the present invention in detail but are not to be construed to limit the scope thereof.

In the examples, aspartase activity was determined by contacting 1 M ammonium fumarate solution containing 1 mM magnesium chloride (pH 8.7) with the microbial cells or the processed material thereof, incubating at 37°C for 15 to 60 minutes to effect the enzymatic reaction, and measuring L-aspartic acid in the resulting reaction mixture by bioassay using Leuconostoc mesenteroides P60 [J. Biol. Chem., 172, 15 (1948)]. D-Serin deaminase activity was determined according to the method described in J. Biol. Chem., 241, 1239 (1966). Citric acid synthase activity was determined according to the method described in Methods in Enzymology, 13, (1969). $\alpha$-Ketoglutarate dehydrogenase activity was determined according to the method described in Seikagaku Jikken Koza, Vol. 12, p 196 (1976). Glutamate dehydrogenase activity was determined according to the method described in J. Microbiol., 64, 187 (1970). Further, fumarase activity was determined as follows:

0.2 M Potassium fumarate-0.01 M potassium phosphate butter (pH 7.0, 30 ml) was added to an enzyme, microbial cells or a processed material of microbial cells and the mixture was reacted with shaking at 37°C for 1 hour. Hydrochloric acid was added to the reaction mixture to remove unreacted fumaric acid. The amount of L-malic acid in the supernatant was colorimetrically determined by reacting it with 2,7-naphthalene diol to develop color.

Example 1

Serratia marcescens SR41 was treated with N-methyl-N'-nitro-N-nitrosoguanidine according to a standard method and 5 x $10^{10}$ microbial cells thus treated were inoculated into a spinner flask (volume 50 ml, manufactured by Belco Co., U.S.A.) containing a medium (50 ml) composed of glucose (3.0 %), sodium L-aspartate (0.015 %), potassium dihydrogen phosphate (0.3 %), dipotassium hydrogen phosphate (0.7 %) and magnesium sulfate heptahydrate (0.01 %). Then, a continuous cultivation was carried out at the degree of dilution of 0.1/hr at 30°C for 8 days. The resulting culture was diluted $10^5$ to $10^6$ times with physiological saline and spread on plates containing a medium having the same composition as the above except that the concentration of sodium L-aspartate was 0.5 % and 1.5 % of agar was added. After cultivation at 30°C for 1 to 2 days, resulting large colonies were picked up to isolate Serratia marcescens APc-494 (FERM BP-392). Aspartase activities and activities of the enzymes responsible for TCA cycle of the microorganism thus obtained and the parent strain were measured. The results are shown in Tables 1 and 2.

Table 1

| Microorganisms | Aspartase activity ($\mu$mole/min/mg protein) | |
|---|---|---|
| | with Glucose (3 %) | without Glucose |
| S. marcescens APc-494 | 10.1 | 13.5 |
| S. marcescens SR41 | 0.60 | 2.70 |

Basal medium: potassium dihydrogen phosphate 0.3 %

- 14 -

0111293

dipotassium hydrogen phosphate 0.7 %, magnesium sulfate heptahydrate 0.01 %, ammonium sulfate 0.1 % and sodium L-aspartate 0.2 %, D-serine 0.2 % (pH 7.0).

As is seen from Table 1, in comparison with the parent strain 8000, the aspartase activity of APc-494 strain is increased about 17 times in the presence of glucose and 5 times in the absence of glucose. In view of this, it is recognized that APc-494 strain is a mutant resulted from mutation of a gene responsible for the production of aspartase as well as release of catabolite repression.

Table 2

| Microorganisms | Enzyme activities ($\mu$mole/min/mg protein) | | | | |
|---|---|---|---|---|---|
| | A | B | C | D | E |
| S. marcescens APc-494 | 1.28 | 0.53 | 2.36 | 0.45 | 0.074 |
| S. marcescens SR41 | 0.14 | 0.12 | 0.25 | 0.09 | 0.012 |

In Table 2, A to E represent the following enzymes: A: D-serine deaminase; B: Glutamate dehydrogenase; C: fumarase; D: citric acid synthase; and E: $\alpha$-ketoglutarate dehydrogenase complex. The basal medium used was the same as that used in Table 1 except that glucose (3 %) was added.

As is seen from Table 2, in comparison with the parent strain 8000, it is clear that not only aspartase activity but also D-serine deaminase, glutamate dehydrogenase, fumarase, citric acid synthase and $\alpha$-ketoglutarate dehydrogenase activities of APc-494 strain are increased.

Example 2

According to the same manner as described in Example 1, _Serratia marcescens_ SR41 treated with N-methyl-N'-nitro-N-nitrosoguanidine was inoculated in a medium (50 ml) composed of sodium L-aspartate (0.1 %), potassium dihydrogen phosphate (0.3 %), dipotassium hydrogen phosphate (0.7 %) and magnesium sulfate heptahydrate (0.01 %) and continuously cultivated for 8 days. The resulting culture was diluted with physiological saline and spreaded on plates containing a medium having the same composition as the above except that concentration of sodium L-aspartate was 0.5 % and 1.5 % of agar was added. After cultivation at 30°C for 1 to 2 days, resulting large colonies were collected to isolate _Serratia marcescens_ APc-109 (FERM BP-391). Aspartase activities and activities of the enzymes responsible for TCA cycle of the microorganism thus obtained and the parent strain, _Serratia marcescens_ SR41 were measured. The results are shown in Tables 3 and 4.

·Table 3

| Microorganisms | Aspartase activity ($\mu$mole/min/mg protein) | |
|---|---|---|
| | with Glucose (3 %) | without Glucose |
| _S. marcescens_ APc-109 | 9.00 | 12.5 |
| _S. narcescens_ SR41 | 0.60 | 2.70 |

Basal medium is the same as that used in Example 1, Table 1.

As is seen from Table 3, in comparison with the parent strain, aspartase activity of APc-109 is increased 15 times in the presence of glucose and about 5 times in the absence of glucose.  In view of this, it is recognized that Serratia marcescens APc-109 strain is a mutant resulted from both mutation of a gene responsible for the production of aspartase and release of catabolite repression.

Table 4

| Microorganisms | Enzyme activities (μmole/min/mg protein) | | | | |
|---|---|---|---|---|---|
| | A | B | C | D | E |
| S. marcescens APc-109 | 0.85 | 0.70 | 2.60 | 0.75 | 0.09 |
| S. marcescens SR41 | 0.14 | 0.12 | 0.25 | 0.09 | 0.012 |

In Table 4, A to E have the same as those in Table 2.  The basal medium is the same as that used in Example 1, Table 2.

As is seen from Table 4, in comparison with the parent strain, it is clear that not only aspartase activity but also D-serine deaminase, glutamate dehydrogenase, fumarase, citric acid synthase and α-ketoglutarate dehydrogenase activities of APc-109 strain are increased.

Example 3

(1)  Serratia marcescens APc-494 was inoculated in a medium (500 ml, pH 7.0) containing ammonium fumarate (3 %), potassium dihydrogen phosphate (0.2 %), magnesium sulfate heptahydrate (0.05 %), corn steep liquor (4 %) and yeast extract (2 %) and cultivated with shaking at 37°C for 24 hours. The resulting culture was centrifuged to collect microbial cells and the cells were suspended in physiological saline (12 ml, wet cells 6 g). To the suspension was added 3.2 % aqueous solution of GENU GEL WG (carrageenan manufactured by Kopenhagen Pectin Factory Ltd.) (48 ml) previously warmed to 37°C and thoroughly stirred. The resulting mixture was added dropwise to 1 M aqueous ammonium fumarate solution containing 1 mM magnesium chloride (pH 8.5) to give immobilized Serratia marcescens (60.6 g, wet weight) having aspartase activity in globular gel (3 mm diameter). The aspartase activity per 1 g of the resulting immobilized cells was 210.6 $\mu$mole/hr.

(2)  The immobilized Serratia marcescens prepared in the above (1) (60 g) was packed in a jacketed column (4. cm x 8 cm) and incubated at 37°C for 48 hours to activate the immobilized cells (aspartase activity: 315,000 $\mu$mole/hr). After activation, 1 M ammonium fumarate solution containing 1 mM magnesium chloride (pH 8.5, 1,000 ml) was passed down through the column at 37°C at the flow rate of 50 ml/hr. The effluent was adjusted to pH 2.8 to give L-aspartic acid (121 g).

Example 4

(1)  The microbial cells of Serratia marcescens APc-494 (8 g) obtained by the same manner as in Example 3 were suspended in physiological saline (5 ml). To the suspension was added 2.2 % aqueous solution of GENU GEL WG containing 1 % locust bean gum (80 ml) previously warmed to 40°C and stirred. The resulting mixture was gelled by cooling. Further, 2 % aqueous potassium chloride solution (250 ml) was slowly added and the mixture was allowed to stand for 30 minutes. The gel was shaped into cubes of 3 mm in side length and washed with 2 % aqueous potassium chloride solution. The gel (89.3 g) thus obtained was dipped in cooled ethanol (100 ml) and added thereto glutaraldehyde in such an amount that the final concentration thereof was 0.49 %. The mixture was allowed to stand with ice-cooling for 15 minutes. Then, the gel was filtered off and washed with 2 % aqueous potassium chloride solution to obtain an immobilized Serratia marcescens (84.4 g, wet weight, aspartase activity: 17,396 $\mu$moles/hr/g cells).

(2) The immobilized Serratia marcescens obtained in the above (1) (11 g) was packed in a jacketed column (1.6 cm x 12 cm) and 1 M aqueous ammonium fumarate solution containing 1 mM magnesium chloride (pH 8.5) was continuously passed through the column at 37°C at the flow rate of 6 ml/hr. Stability of the immobilized cells was evaluated by measuring the aspartase activity of samples of the effluent with time. As the result, even after 14 days, decrease of the activity was scarcely observed.

Example 5

The microbial cells of Serratia marcescens APc-494 obtained by the same manner as in Example 3 (10 g) was suspended in 0.05 M phosphate buffer (pH 8.5, 50 ml) and sonicated at 9 Kc for 10 minutes. Then, the suspension was centrifuged and the resulting supernatant (35 ml) was passed through a column packed with Duolite-A7 (a weak basic ion exchange resin manufactured by Diamond Shamrock Chemical Co. in U.S.A.) (60 ml) at room temperature at SV = 0.75. Then, the column was treated with a solution containing 0.1 M phosphate buffer (pH 8.5, 300 ml) and 0.4 % glutaraldehyde (300 ml) and excess glutaraldehyde was thoroughly washed out to give an immobilized enzyme. The immobilized enzyme was packed in a column (volume 50 ml) and 1 M ammonium fumarate solution containing 1 mM magnesium chloride (pH 8.5, 500 ml) was passed through the column at the flow rate of 25 ml/hr. The combined effluent was adjusted to pH 2.8 to precipitate crystals. The crystals were filtered off to give L-aspartic acid (58 g).

Example 6

According to the same manner as in Example 5, a supernatant was obtained by using Serratia marcescens APc-494. The supernatant was subjected to ammonium sulfate fractionation (saturation degree: 30 to 50 %) and the resulting precipitate was dissolved in water (5 ml). The solution was dialyzed overnight against water and the resulting dialysate was taken as an enzyme solution (standard aspartase). Then, the enzyme solution (2 ml) and 3.2 % aqueous GENU GEL WG solution (12 ml) were mixed in a water bath at 37°C. The mixture was added dropwise to 2 % aqueous potassium chloride solution to give globular gel (about 3 mm diameter). The aspartase activity of the resulting immobilized aspartase was 4,786 $\mu$moles/hr/g.

Example 7

Serratia marcescens APc-494 was inoculated in the same medium (1 liter) as that used in the continuous cultivation in Example 1 except that the concentration of sodium L-aspartate was 1.0 % and cultivated with shaking at 37°C for 10 hours. The resulting culture was centrifuged to collect microbial cells and the cells were suspended in physiological saline (12 ml, wet cells 6 g). To the suspension was added 3.2 % aqueous GENU GEL WG solution previously warmed to 37°C and thoroughly mixed. The mixture was added dropwise to 1 M ammonium fumarate solution containing 1 mM magnesium chloride (pH 8.5) to obtain an immobilized Serratia marcescens (60 g, wet weight) in globular gel (about 3 mm diameter) having aspartase activity. The aspartase activity per 1 g of the immobilized cells were 4,786 $\mu$ mole/hr/g.

Example 8

(1)  Serratia marcescens APc-109 was inoculated in the medium (1 liter) as that used in the continuous cultivation in Example 2 except that the concentration of sodium L-aspartate was 1.0 % and cultivated with shaking at 30°C for 10 hours. By using the microbial cells thus obtained, according to the same manner as in Example 7, an immobilized Serratia marcescens (60 g, wet weight) was prepared. The aspartase activity of the immobilized cells were 16.8 $\mu$ mole/hr/g.

(2)  The above-obtained immobilized Serratia marcescens (60 g) was packed in a jacketed column (4 cm x 8 cm) and incubated at 37°C for 48 hours to activate the immobilized cells.  After activation (aspartase activity: 30,240 μmoles/hr), 1 M ammonium fumarate solution containing 1 mM magnesium chloride (pH 8.5, 1,000 ml) was passed down through the column at 37°C at the flow rate of 50 ml/hr.  The effluent was adjusted to pH 2.8 to precipitate crystals.  The crystals were filtered off to give L-aspartic acid (122 g).

Example 9

(1)  Serratia marcescens APc-494 was inoculated in a medium (500 ml, pH 7.0) containing ammonium fumarate (3 %), potassium dihydrogen phosphate (0.2 %), magnesium sulfate heptahydrate (0.05 %), corn steep liquor (4 %) and yeast extract (2 %) and cultivated at 37°C for 24 hours. The resulting culture was adjusted to pH 8.5 with addition of aqueous ammonia and added thereto ammonium fumarate (65 g) and Triton X-100 (500 mg).  The mixture was allowed to stand at 37°C for 48 hours.  Then, the mixture was adjusted to pH 2.3 to precipitate crude crystals of L-aspartic acid. The crude crystals were filtered off and recrystallized from water to give L-aspartic acid (46.0 g).

CLAIMS:

1. A microorganism of the genus Serratia for use in producing L-aspartic acid which has high aspartase activity and is released from catabolite repression.

2. A microorganism of claim 1 being Serratia marcescens APc-109 (FERM BP-391).

3. A microorganism of claim 1 being Serratia marcescens APc-494 (FERM BP-392).

4. A method for producing a microorganism of the genus Serratia for use in producing L-aspartic acid having high aspartase activity and being released from catabolite repression which comprises mutagenizing a parent strain of the genus Serratia having aspartase activity and being subjected to catabolite repression, cultivating the treated microorganism in a medium containing L-aspartic acid either as a sole nitrogen source or as sole carbon and nitrogen sources and isolating a microorganism being able to grow in the medium.

5. A method of claim 4, wherein the parent strain is Serratia marcescens having aspartase activity and being subjected to catabolite repression.

6. A method of claim 5, wherein the microorganism produced is Serratia marcescens APc-109 (FERM BP-391).

7. A method of claim 5, wherein the microorganism produced is Serratia marcescens APc-494 (FERM BP-392).

8. A method for producing L-aspartic acid which comprises contacting

(a) a culture of a microorganism of the genus Serratia having high aspartase activity and being released from catabolite repression which has obtained by mutagenizing a parent strain having aspartase activity and being subjected to catabolite repression, cultivating the treated microorganism in a medium containing L-aspartic acid either as a sole nitrogen source or as sole carbon and nitrogen sources and isolating a microorganism being able to grow in the medium;

(b) microbial cells collected from the culture; or

(c) a processed material of the microbial cells, with fumaric acid and ammonia to produce L-aspartic acid and then collecting L-aspartic acid thus produced.

9. A method according to claim 8, wherein the parent strain is Serratia marcescens having aspartase activity and being subjected to catabolite repression.

10. A method according to claim 9, wherein the microorganism obtained from the parent strain is Serratia marcescens having high aspartase activity and being released from catabolite repression.

11. A method according to claim 10, wherein the microorganism is Serratia marcescens APc-109 (FERM BP-391).

12. A method according to claim 10, wherein the microorganism is Serratia marcescens APc-494 (FERM BP-392).

13. A method according to claim 8, wherein fumaric acid and ammonia is used in the form of ammonium fumarate.

14. A method according to claim 8, wherein the contact is effected at about 5 to 50°C at pH of 6 to 10.